# EUROPÄISCHE PATENTSCHRIFT

(11) **EP 0 987 002 B1**
(45) Veröffentlichungstag und Bekanntmachung des Hinweises auf die Patenterteilung: **22.11.2006**
(21) Anmeldenummer: 99116870.9
(22) Anmeldetag: 06.09.1999
(51) Int. Cl.: A61Q 1/02, A61Q 1/14, A61Q 15/00, A61Q 17/04, A61Q 19/00, A61K 8/25, A61K 8/27, A61K 8/29, A61K 8/37

(54) **Emulgatorfreie feindisperse Systeme vom Typ Öl-in-Wasser und Wasser-in-Öl**
Emulsifier-free finely dispersed systems of the oil-in-water or water-in-oil type
Systèmes en dispersion fine, sans émulsifiants, de type huile-dans-eau ou eau-dans-huile

(30) Priorität: 18.09.1998 DE 19842730
(43) Veröffentlichungstag der Anmeldung: 22.03.2000
(73) Patentinhaber: Beiersdorf Aktiengesellschaft, 20245 Hamburg (DE)
(72) Erfinder: Gers-Barlag, Heinrich, Dr., 25495 Kummerfeld (DE); Müller, Anja, 23843 Rümpel (DE)

(56) Entgegenhaltungen:
- EP-A- 0 456 460
- EP-A- 0 610 926
- DE-A- 4 425 268
- FR-A- 2 686 510
- US-A- 5 804 167

## Beschreibung

Die vorliegende Erfindung betrifft emulgatorfreie feindisperse Systeme vom Typ Öl-in-Wasser und Wasser-in-Öl, bevorzugt als kosmetische oder dermatologische Zubereitungen.

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze ÖIIWasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Ver bindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder lsofettsäuren.
Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

Kosmetische Zubereitungen werden im wesentlichen zur Hautpflege benutzt. Hautpflege im kosmetischen Sinn ist in erster Linie, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Kosmetische Zubereitungen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Medizinische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, daß bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzlich stabilisierende Wirkung haben. Dabei kommt es zu einer Anreicherung des festen Stoffes an der Phasengrenze Öl/Wasser in Form einer Schicht, wodurch Zusammenfließen der dispersen Phasen verhindert wird. Von wesentlicher Bedeutung sind dabei nicht die chemischen, sondern die Oberflächeneigenschaften der Feststoffpartikel.

Pickering stellte um 1910 erstmals Paraffin-Wasser-Emulsionen her, die nur durch den Zusatz verschiedener Feststoffe wie basisches Kupfersulfat, basisches Eisensulfat oder andere Metallsulfate stabilisiert wurden. Diese Art von Emulsionen wird daher auch als Pickering-Emulsion bezeichnet.

Sozusagen die Urformen von Pickering-Emulsionen tauchten zunächst als unerwünschte Nebeneffekte bei unterschiedlichen technischen Prozessen auf, wie z. B. bei der sekundären Erdölförderung, der Extraktion von Bitumen aus Teersand und anderen Trennungsverfahren, an denen zwei nicht miteinander mischbare Flüssigkeiten und feine, dispergierte Feststoffpartikel beteiligt sind. Im allgemeinen handelt es sich hierbei um W/O-Emulsionen, die durch mineralische Feststoffe stabilisiert werden. Dementsprechend stand die Untersuchung entsprechender Systeme, wie z. B. der Systeme Öl-Wasser-Ruß oder ÖI-Wasser-Schieferstaub zunächst im Mittelpunkt der Forschungsaktivitäten.

Grundlegende Untersuchungen haben dabei gezeigt, daß ein Charakteristikum für eine Pickering-Emulsion ist, daß die Feststoffpartikel an der Grenzfläche zwischen den beiden flüssigen Phasen angeordnet sind und dort sozusagen eine mechanische Barriere gegen die Vereinigung der Flüssigkeitströpfen bilden.

Eine relativ neue technische Entwicklung ist es, Pickering-Emulsionen als Grundlage für kosmetische oder dermatologische Zubereitungen zu verwenden.

Eine Möglichkeit, eine Feststoffstabilisierung im Sinne einer Pickering-Emulsion in einer kosmetischen oder dermatologischen Zubereitung vorzunehmen, ist nach May-Alert (*Pharmazie in unserer Zeit, 15. Jahrg. 1986, Nr. 1, 1-7*) beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung erreichen.

Darüber hinaus beschreibt die Europäische Offenlegungsschrift 0 686 391 Emulsionen vom Typ Wasser-in-Öl, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 µm haben. Diese Emulsionen können nach dem oben gesagten als Pickering-Emulsionen bezeichnet werden.

Pickering-Emulsionen werden durch den Einsatz von geeigneten Feststoffen bzw. Pigmenten stabilisiert. Allerdings haben die Zubereitungen des Standes der Technik im allgemeinen den Nachteil, daß sie auf einen engen Anwendungsbereich oder eine begrenzte Einsatzstoffauswahl begrenzt sind, da sie sich nur auf diese Weise stabil formulieren lassen. Für einige Bereiche der Kosmetik (z. B. für den Bereich der Gesichtspflege) weisen Pickering-Emulsionen des Standes der Technik unbefriedigende kosmetische Eigenschaften auf.

Aufgabe war daher, den Nachteilen des Standes der Technik Abhilfe zu schaffen. Insbesondere sollten kosmetische und dermatologische Grundlagen für kosmetische und dermatologische Zubereitungen zur Verfügung gestellt werden, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen oder Deodorantien, aber auch ausgesprochen medizinisch-pharmazeutische Darreichungsformen geschaffen werden, zum Beispiel Zubereitungen gegen Akne und andere Hauterscheinungen.

Es war erstaunlich und für den Fachmann in keiner Weise vorauszusehen, daß Pickering-Emulsionen, welche feindisperse Systeme vom Typ Wasser-in-Öl oder Öl-in-Wasser darstellen, enthaltend
(1) eine Ölphase, die mindestens ein Wachs enthält, welches aus der Gruppe der Triglyceride gewählt wird,
(2) eine Wasserphase,
(3) mindestens einen Typ mikrofeiner Partikel, die
   a) eine mittlere Partikelgröße von weniger als 200 nm haben, die
   b) sowohl hydrophile als auch lipophile Eigenschaften zeigen, welche also amphiphilen Charakter besitzen und sowohl in Wasser als auch in Öl dispergierbar sind und die
   c) gegebenenfalls oberflächlich beschichtet sind und
(4) höchstens 0,5 Gew.-% eines oder mehrerer Emulgatoren
den Nachteilen des Standes der Technik abhelfen.

Es ist erfindungsgemäß besonders vorteilhaft, wenn die Zubereitungen deutlich weniger als 0,5 Gew.-% eines oder mehrerer Emulgatoren enthalten bzw. sogar gänzlich emulgatorfrei sind.

Die erfindungsgemäßen Zubereitungen sind in jeglicher Hinsicht überaus befriedigende Präparate, die gegenüber herkömmlichen Pickering-Emulsionen eine deutlich höhere Stabilität haben und daher insbesondere geeignet sind, um als Grundlage für Zubereitungsformen mit vielfältigen Anwendungszwecken zu dienen. Insbesondere W/O-Pickering-Emulsionen im Sinne der vorliegenden Erfindung sind erstaunlicherweise ausgesprochen stabil.

Zudem zeichnen sich die erfindungsgemäßen Zubereitungen durch eine ausgezeichnete Hautverträglichkeit aus. Ferner war erstaunlich, daß erfindungsgemäße Zubereitungen, die in der Form eines Sonnenschutzmittels vorliegen, eine höhere Wirksamkeit zeigen als übliche Sonnenschutzformulierungen.

Während Zubereitungen des Standes der Technik bereits mit einem Pigmentgehalt von 1 Gew.-% ein stumpfes Gefühl nach ihrer Anwendung auf der Haut erzeugen, das mit höheren Pigmentkonzentrationen noch zunimmt, hinterlassen die erfindungsgemäßen Zubereitungen überraschend keinen trockenen oder stumpfen Eindruck auf der Haut, sondern zeigen vielmehr hervorragende kosmetische Eigenschaften.

Zwar kennt der Stand der Technik neben Pickering-Emulsionen emulgatorfreie, feindisperse kosmetische oder dermatologische Zubereitungen, die im allgemeinen als Hydrodispersionen bezeichnet werden und welche Dispersionen einer flüssigen, halbfesten oder festen inneren (diskontinuierlichen) Lipidphase in einer äußeren wäßrigen (kontinuierlichen) Phase darstellen. Allerdings konnte der Stand der Technik nicht den Weg zur vorliegenden Erfindung weisen.

Bei Hydrodispersionen einer flüssigen Lipidphase in einer äußeren wäßrigen Phase kann die Stabilität beispielsweise dadurch gewährleistet werden, daß in der wäßrigen Phase ein Gelgerüst aufgebaut wird, in welchem die Lipidtröpfchen stabil suspendiert sind.

Die Deutsche Offenlegungsschrift 44 25 268 beschreibt stabile feindisperse, emulgatorfreie kosmetische oder dermatologische Zubereitungen vom Typ Öl-in-Wasser, die neben einer Öl- und einer Wasserphase einen oder mehrere Verdicker aus der Gruppe der Acrylsäurepolymere, Polysaccharide und deren Alkylether enthalten, wobei für diese Verdicker eine Grenzflächenspannungserniedrigung nicht meßbar sein darf.

Basierend auf ähnlichen Hydrodispersionen werden in der Deutschen Offenlegungsschrift 43 03 983 kosmetische oder dermatologische Lichtschutzformulierungen offenbart, die im wesentlichen frei von Emulgatoren sind, wobei in die Lipidphase der Hydrodispersion anorganische Mikropigmente eingearbeitet sind, die als UV-Filtersubstanzen dienen.

O/W-Pickering-Emulsionen im Sinne der vorliegenden Erfindung hingegen sind erhältlich, indem man erfindungsgemäße, für die Herstellung von O/W-Pickering-Emulsionen geeignete amphiphile Partikel zunächst in der Wasserphase dispergiert und die Wasserphase anschließend mit der Fettphase vereinigt. Erfindungsgemäße W/O-Pickering-Emulsionen sind dagegen durch Dispergieren von erfindungsgemäßen, für die Herstellung von W/O-Pickering-Emulsionen geeigneten amphiphilen Partikeln in der Fettphase erhältlich.

### Wachse:

Nach Festlegung der Deutschen Gesellschaft für Fettwirtschaft (*Fette, Seifen, Anstrichmittel, **76**, 135 [1974])* werden zur Kennzeichnung des Begriffes "Wachs" in der Regel die mechanisch-physikalischen Eigenschaften der Wachse, welche für ihre Verwendung maßgebend sind, herangezogen, während für die Begriffsbestimmung die jeweilige chemische Zusammensetzung unberücksichtigt bleibt.

"Wachs" ist - ähnlich wie "Harz" - eine Sammelbezeichnung für eine Reihe natürlicher oder künstlich gewonnener Stoffe, die in der Regel folgende Eigenschaften aufweisen: bei 20 °C knetbar, fest bis brüchig hart, grob- bis feinkristallin, durchscheinend bis opak, jedoch nicht glasartig, über 40 °C ohne Zersetzung schmelzend, schon wenig oberhalb des Schmelzpunkts verhältnismäßig niedrigviskos und nicht fadenziehend, stark temperaturabhängige Konsistenz und Löslichkeit und unter leichtem Druck polierbar. Ist in Grenzfällen bei einem Stoff mehr als eine der vorstehend genannten Eigenschaften nicht erfüllt, so ist er kein Wachs im Sinne dieser Definition. Wachse unterscheiden sich von ähnlichen synthetischen oder natürlichen Produkten (z. B. Harzen, plastischen Massen usw.) hauptsächlich darin, daß sie in der Regel etwa zwischen 50 und 90 °C, in Ausnahmefällen auch bis zu etwa 200 °C, in den schmelzflüssigen, niedrigviskosen Zustand übergehen und praktisch frei von aschebildenden Verbindungen sind.

Wachses im Sinne der vorliegenden Erfindung sind Verbindungen, welche dadurch charakterisiert sind, daß sie zusammen mit den anderen Ölkomponenten der erfindungsgemäßen Zubereitungen (wie beispielsweise polare, flüssige Verbindungen, UV-Filter und deren Lösungsmittel usw.) eine bei Raumtemperatur pastöse (dickflüssige) und streichfähige Masse bilden, welche bei 20 °C eine Viskosität von mehr als 5000 mPa·s aufweist.

Erfindungsgemäß vorteilhaft sind z. B. natürliche Wachse tierischen und pflanzlichen Ursprungs, wie beispielsweise Bienenwachs, Chinawachs, Hummelwachs und andere Insektenwachse, insbesondere die nachstehend genannten.

Bienenwachs z. B. ist ein Ausscheidungsprodukt aus Drüsen der Honigbienen, das diese zum Bauen der Honigwaben verwenden. Gelbes (Cera flava), braunes oder rotes sogenanntes Rohwachs ist beispielsweise erhältlich, indem man die vom Honig durch Ausschleudern befreiten Waben schmilzt, die Schmelze von festen Verunreinigungen trennt und das so erhaltene Rohwachs erstarren läßt. Das Rohwachs kann durch Behandlung mit Oxidationsmitteln vollkommen weiß gebleicht werden (Cera alba).

Bienenwachs besteht aus dem in Alkohol leichtlöslichen Cerin, einem Gemisch aus Cerotinsäure CH₃(CH₂)₂₄COOH und Melissinsäure CH₃(CH₂)₂₈COOH sowie aus einem Myricin genannten Ester-Gemisch aus ca. 70 Estern von C₁₆- bis C₃₆-Säuren und C₂₄₋bis C₃₆-Alkoholen. Als wesentliche Bestandteile von Bienenwachs finden sich Myricylpalmitat, Myricylcerotinat und Paraffin.

Auch andere Insektenwachse wie beispielsweise Hummelwachs, Schellackwachs oder Chinawachs sind im wesentlichen Mischungen verschiedener Ester. Chinawachs z. B. wird in China und Japan von der auf der chinesischen Esche lebenden Wachsschildlaus (Coccus ceriferus) und den Schildlausarten Ceroplastes ceriferus und Ericerus pela abgeschieden bzw. erzeugt. Es wird von den Bäumen abgekratzt und durch Umschmelzen in kochendem Wasser gereinigt. Hauptbestandteil des Chinawachses ist der Cerotinsäureester des Cerylalkohols.

Schellackwachs wird aus Lac gewonnen, dem Sekret der weiblichen Lackschildläuse (Kerria lacca), welche in riesigen Kolonien (Lac ist abgeleitet von dem Hindhi-Wort "Lakh" für 100 000) auf Bäumen und Sträuchern im südasiatischen Raum (Indien, Burma, Südchina) leben. Das durch Lösemittel-Extraktion zugängliche Schellackwachs enthält als wesentliche Bestandteile Myricylalkohol, Melissinsäure und andere Wachsalkohole und -säuren bzw. deren Ester.

Auch Pflanzenwachse sind vorteilhaft im Sinne der vorliegenden Erfindung. Vorzugsweise verwendbar sind Cuticularwachse niederer und höherer Pflanzen, Algen, Flechten, Moose und Pilze, wie beispielsweise Candelillawachs, Carnaubawachs, Japanwachs, Espartograswachs, Korkwachs, Reiswachs, Zuckerrohrwachs, Fruchtwachse, z. B. Apfelwachs, Blütenwachse, Blattwachse von Nadelhölzern, Kaffeewachs, Flachswachs, Sesamwachs, Jojobaöl und dergleichen mehr.

Candelillawachse z. B. sind bräunliche bis gelblichbraune, harte wachsartige Massen, welche in lipophilen Lösemitteln löslich sind. Candelillawachs enthält ungeradzahlige aliphatische Kohlenwasserstoffe (ca. 42 %), Ester (ca. 39 %), Wachssäuren und Wachsalkohole. Gewonnen werden kann es beispielsweise aus den zerkleinerten, fleischigen Blättern einer stachellosen Wolfsmilchart (Euphorbia cerifera) durch Auskochen mit wäßriger Schwefelsäure.

Carnaubawachs ist eine gelbliche, grünliche oder dunkelgraue Masse, welche in verschiedenen durch Auslese gewonnenen Qualitäten aus den Blättern der brasilianischen Fächerpalme Copernicia prunifera oder Camaubapalme (Camauba cerifera) gewonnen werden kann, indem beispielsweise der Wachsstaub von den angewelkten Wedeln gebürstet, geschmolzen, filtriert und nach dem Festwerden in Stücke gebrochen wird. Camaubawachs kann durch Bleichmittel aufgehellt werden. Es enthält ca. 85 % Ester, jeweils etwa 2-3 % freie Wachssäuren (Camauba-, Behen-, Lignocerin-, Melissin- und Cerotinsäure), langkettige Alkohole, Diole und gesättigte Kohlenwasserstoffe.

Japanwachs (auch: Japantalg oder Cera japonica) ist farbloses oder gelbliches, reines Pflanzenfett, das beispielsweise in Japan aus den Früchten eines baumförmigen Sumach-Gewächses (Rhus succedanea) durch Auskochen gewonnen werden kann. Hauptbestandteile des Japanwachses sind Palmitinsäureglycerinester sowie Ester der Japansäure (Heneicosandisäure, C₂₁H₄₀O₄), der Phellogensäure (Docosandisäure, C₂₂H₄₂O₄) und der Tricosandisäure (C₂₃H₄₄O₄).

Esparto-Wachs fällt als Nebenprodukt bei der Zellstoff- und Papierherstellung aus dem in Mittelmeerländern beheimateten Espartogras (Graminaceae) an. Es zu besteht zu ca. 15 bis 17 % aus Wachssäuren (z. B. Cerotin- und Melissinsäure), zu 20 bis 22 % aus Alkoholen und Kohlenwasserstoffen sowie zu 63 bis 65 % aus Estern.

Insbesondere vorteilhaft im Sinne der vorliegenden Erfindung sind beispielsweise die unter den Handelbezeichnungen Permulgin 1550 und Permulgin 4002 bei KOSTER KEUNEN und die unter den Handelbezeichnungen Schellack Wachs 7302 L und Candellila Wachs 2039 L bei der KAHL Wachsraffinerie erhältlichen natürlichen Wachse.

Erfindungsgemäß vorteilhaft sind ferner chemisch modifizierte Wachse und synthetische Wachse. Bevorzugte modifizierte Wachse sind beispielsweise Bienenwachsester, insbesondere die unter den Handelsnamen BW Ester BW 67, BW Ester BW 80 bei KOSTER KEUNEN erhältlichen Alkylbienenwachse.

Bevorzugte synthetische Wachse sind beispielsweise das unter der Handelsbezeichnung Bienenwachskomponete B 85 bei SCHLICKUM erhältliche sowie Wachse auf Silikonbasis wie z. B. Dialkoxydimethylpolysiloxane, welche sich durch die folgende Struktur auszeichnen 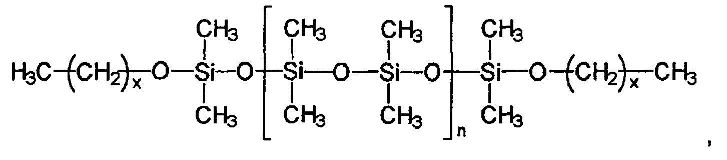 worin x eine Zahl zwischen 18 und 24 bedeutet. Insbesondere vorteilhaft ist das Behenoxy Dimethicon, für welches x aus der obigen Strukturformel 21 bedeutet und welches unter der Handelsbezeichnung Abil^{®} Wax 2440 bei der Th. Goldschmidt AG erhältlich ist. Erfindungsgemäß bevorzugt ist ferner ein Wachs auf Silikonbasis, das unter der Handelsbezeichnung Siliconyl Beeswax bei KOSTER KEUNEN erhältlich ist.

Weitere vorteilhafte synthetische Wachse sind bestimmte Fettsäuren und/oder Fettsäuremischungen, beispielsweise C₁₆₋₃₆-Fettsäuren, insbesondere solche, die unter der Handelsbezeichnung Syncrowax AW1 C bei Croda GmbH erhältlich sind.

Vorteilhaft im Sinne der vorliegenden Erfindung sind außerdem Esterwachse, die Ester aus
1. einer gesättigten und/oder ungesättigten, verzweigten und/oder unverzweigten Mono- und/oder Dicarbonsäure mit 12 bis 40 Kohlenstoffatomen und
2. einem gesättigten und/oder ungesättigten, verzweigten und/oder unverzweigten Alkohol mit 12 bis 40 Kohlenstoffatomen darstellen.
Insbesondere vorteilhaft sind Esterwachse, die gewählt werden aus der im folgenden aufgelisteten Gruppe:

| **Esterwachs** | **Handelsname** | **erhältlich bei** |
|---|---|---|
| Myristylmyristat | Cetiol MM | Henkel KGaA |
| Cetylpalmitat | Cutina CP | Henkel KGaA |
| C₁₄₋₃₄ Alkylstearat | Kesterwachs K 76 H | KOSTER KEUNEN |
| C₂₀₋₄₀ Dialkyldimerat | Kesterwachs K 80 D | KOSTER KEUNEN |
| Ditetracosanyldimerat | Kesterwachs K70D | KOSTER KEUNEN |
| C₁₆₋₃₈ Alkylhydroxystearoylstearat | Kesterwachs K80P | KOSTER KEUNEN |
| C₂₀₋₄₀ Alkylstearoylstearoylstearat | Kesterwachs K80P-VS | KOSTER KEUNEN |
| C₂₀₋₄₀ Alkylstearat | Kesterwachs K 82 | KOSTER KEUNEN |
| Hydroxystearylhydroxystearat | Elfacos C26 | AKZO NOBEL |

Weiterhin vorteilhaft sind Ester des Glykols, insbesondere Glykolester der Lignocerinsäure (CH₃(CH₂)₂₂COOH), der Cerotinsäure (CH₃(CH₂)₂₄COOH) und/oder der Montansäure (CH₃(CH₂)₂₆COOH). Ganz besonders vorteilhaft im Sinne der vorliegenden Erfindung sind Glykolester der Montansäure (CH₃(CH₂)₂₆COOH). Ein vorteilhaftes Glykolmontanat ist z.B. in einer Mischung mit Butylenglykolmontanat unter der Handelsbezeichnung Wax E Pharma bei der Firma Clariant erhältlich.

Es ist ferner vorteilhaft, die Wachskomponenten aus der Gruppe der Glyceride, insbesondere aus der Gruppe der Triglyceride zu wählen. Besonders vorteilhaft sind die im folgenden aufgelisteten Glyceride und Triglyceride:

| **Glycerid** | **Handelsname** | **erhältlich bei** |
|---|---|---|
| C₁₆₋₁₈ Triglycerid | Cremeol HF-52-SPC | Aarhus Oliefabrik |
| Glycerylhydroxystearat | Naturchem GMHS | Rahn |
| Hydrierte Coco-Glyceride | Softisan 100 | Hüls AG |
| Caprylic/Capric/Isostearic/Adipic Triglycerid | Softisan 649 | Dynamit Nobel |
| C₁₈₋₃₆ Triglycerid | Syncrowax HGLC | Croda GmbH |
| Glyceryltribehenat | Syncrowax HRC | Croda GmbH |
| Glyceryl-tri-(12-hydroxystearat) | Thixcin R | Rheox / N RC |
| Hydriertes Ricinusöl | Cutina HR | Henlek KGaA |
| C₁₆₋₂₄ Triglycerid | Cremeol HF-62-SPC | Aarhus Oliefabrik |

Besonders bevorzugt im Sinne der vorliegenden Erfindung ist auch Sheabutter, auch Karitéfett oder Galambutter genannt (CAS-Nr. 68920-03-6). Sheabutter ist das Fett der Samen bzw. Kerne der Familie der Sapotaceae angehörenden Pflanze Butyrospermum Parkii, das zu etwa 34 bis 45 Gew.-% aus festen Fettsäuren (vornehmlich Stearinsäure) und zu etwa 50 bis 60 Gew.-% aus flüssigen Fettsäuren (vornehmlich Ölsäure enthaltend) besteht.

Die Wachse werden erfindungsgemäß ferner vorzugsweise aus der Gruppe der gesättigten und/oder ungesättigten, verzweigten und/oder unverzweigten Fettalkohole mit 14 bis 40 Kohlenstoffatomen gewählt, besonders bevorzugt sind Behenylalkohol (C₂₂H₄₅OH), Cetearylalkohol [eine Mischung aus Cetylalkohol (C₁₆H₃₃OH) und Stearylalkohol (C₁₈H₃₇OH)], Cetylarachidol [2-Hexadecyl-1-Eicosanol (C₃₆H₇₃OH),] und/oder 2-Tetradecyloctadecanol (C₃₂H₆₅OH). Vorteilhafte Ausführungsformen der beiden letztgenannten Fettalkohole sind unter den Handelsnamen Isofol 36 und Isofol 32 bei Condea erhältlich.

Die Gesamtmenge an einem oder mehreren Wachsen in den fertigen kosmetischen oder dermatologischen Zubereitungen wird vorteilhaft aus dem Bereich von 0,5 bis 20,0 Gew.-%, bevorzugt zwischen 1,0 und 5,0 Gew.-% gewählt, bezogen auf das Gesamtgewicht der Zubereitungen.

Es ist ferner vorteilhaft, wenngleich nicht zwingend, wenn die erfindungsgemäßen Pikkering-Emulsionen weitere Hilfsstoffe enthalten, die zur Verminderung oder Verhinderung eines stumpfen oder trockenen Hautgefühls nach deren Auftragen beitragen können, wobei der hauptsächliche Zweck dieser Stoffe ein anderer sein kann. Vorzugsweise werden diese Stoffe beispielsweise gewählt aus der Gruppe der unsymmetrisch substituierten s-Triazinderivate, der Cyclodextrine, der Filmbildner und der polymeren Moisturizer, wobei diese Stoffe sowohl einzeln als auch im Gemisch vorliegen können.

Die kosmetischen Eigenschaften der erfindungsgemäßen Pickering-Emulsionen können zusätzlich beispielsweise noch dadurch verbessert werden, daß in der Ölphase auch Öle eingesetzt werden, welche eine Viskosität haben, die kleiner als 30 mPa·s, insbesondere kleiner als 20 mPa·s ist (bestimmt mit einem Rheometer der Firma Contraves (Rheomat 108E) bei einem Schergefälle von 500/s und einer Temperatur von 25 °C).

### Mikrofeine Partikel:

Der amphiphile Charakter der erfindungsgemäßen mikrofeinen Partikel zeigt sich beispielsweise darin, daß diese sowohl in Wasser als auch in Öl dispergierbar sind.

Es ist vorteilhaft, den mittleren Partikeldurchmesser der verwendeten Partikel zwischen 1 nm und 200 nm, besonders vorteilhaft zwischen 5 nm und 100 nm zu wählen.

Es ist ferner vorteilhaft, die Konzentration aller erfindungsgemäßen amphiphilen Partikel größer als 0,1 Gew.-%, besonders vorteilhaft zwischen 0,1 Gew.-% und 30 Gew.-%, bezogen auf das Gesamtgewicht der Zubereitungen, zu wählen.

Vorteilhaft im Sinne der vorliegenden Erfindung sind alle Partikel, die geeignet sind, Pickering-W/O-Emulsionen bzw. Pickering-O/W-Emulsionen zu stabilisieren. Im wesentlichen unerheblich für die vorliegende Erfindung ist es, in welcher der gegebenenfalls natürlich vorkommenden Modifikationen die Partikel vorliegen.

Vorzugsweise werden zur Stabilisierung der Pickering-Emulsionen unbehandelte, nahezu reine Pigmentpartikel verwendet, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Pickering-Emulsionen im Sinne der vorliegenden Erfindung werden ebenfalls vorteilhaft durch anorganische Pigmente stabilisiert, die oberflächlich wasserabweisend behandelt ("gecoatet") sind, wobei gleichzeitig der amphiphile Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid, als auch durch Kombination mehrerer Pigmenttypen innerhalb einer Zubereitung.

Vorzugsweise werden die erfindungsgemäßen Pickering-Emulsionen ferner durch Bornitridpartikel stabilisiert, beispielsweise durch die im folgenden aufgelisteten Bornitride:

| **Handelsname** | **erhältlich bei** |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN^{®} | Carborundum |
| Wacker-Bornitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Pickering-Emulsionen im Sinne der vorliegenden Erfindung werden ebenfalls vorteilhaft durch Bornitridpartikel stabilisiert, die oberflächlich wasserabweisend behandelt ("gecoatet") sind, wobei gleichzeitig der amphiphile Charakter gebildet werden bzw. erhalten bleiben soll.

Eine vorteilhafte Beschichtung der Bornitridpartikel besteht aus Dimethylpolysiloxan (Dimethicon). Vorteilhaft sind beispielsweise die bei der Firma Carborundum unter der Handelsbezeichnung Très BN^{®} UHP 1106 erhältlichen, mit Dimethicon behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN^{®} UHP 1107 erhältlichen.

Es ist ferner vorteilhaft, die erfindungsgemäßen Pickering-Emulsionen durch mikrofeine Polymerpartikel zu stabilisieren.

Vorteilhafte mikrofeine Polymerpartikel sind im Sinne der vorliegenden Erfindung beispielsweise Polycarbonate, Polyether, Polyethylen, Polypropylen, Polyvinylchlorid, Polystyrol, Polyamide, Polyacrylate und dergleichen mehr.

Erfindungsgemäß vorteilhaft sind beispielsweise mikrofeine Polyamid-Partikel, welche unter der Handelsbezeichnung SP-500 bei der Firma TORAY erhältlich sind. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Launnlactam. Vorteilhaft im Sinne der vorliegenden Erfindung sind beispielsweise Orgasol^{®} 1002 (Polyamid 6) und Orgasol^{®} 2002 (Polyamid 12) von der Firma ELF ATO-CHEM.

Weitere vorteilhafte Polymerpartikel sind mikrofeine Polymethacrylate. Solche Partikel sind beispielsweise unter der Handelsbezeichnung POLYTRAP^{®} bei der Firma DOW CHEMICAL erhältlich.

Es ist insbesondere vorteilhaft, wenngleich nicht zwingend, wenn die verwendeten mikrofeinen Polymerpartikel oberflächlich beschichtet sind. Diese Oberflächenbehandlung kann darin bestehen, daß die Pigmente nach an sich bekannten Verfahren mit einer dünnen hydrophilen Schicht versehen werden. Vorteilhafte Beschichtungen bestehen beispielsweise aus TiO₂, ZrO₂ oder auch weiteren Polymeren, wie beispielsweise Polymethylmethacrylat.

Besonders vorteilhafte mikrofeine Polymerpartikel im Sinne der vorliegenden Erfindung sind ferner nach dem in der US-Patentschrift 4,898,913 beschriebenen Verfahren zur hydrophilen Beschichtung hydrophober Polymerpartikel erhältlich.

Vorteilhaft in ist es, den mittleren Partikeldurchmesser der verwendeten mikrofeinen Polymerpartikel kleiner als 100 µm, besonders vorteilhaft kleiner als 50 µm zu wählen. Dabei ist es im wesentlichen unerheblich, in welcher Form (Plättchen, Stäbchen, Kügelchen etc.) die verwendeten Polymerpartikel vorliegen.

Desweiteren ist es vorteilhaft, die erfindungsgemäßen Pickering-Emulsionen durch modifizierte Polysaccharide zu stabilisieren.

Modifizierte Polysaccharide im Sinne der vorliegenden Erfindung sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Solche Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel 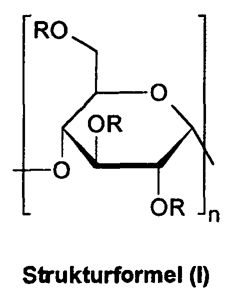 worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhafte Stärkeether sind z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und deren Salze, beispielsweise die Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet 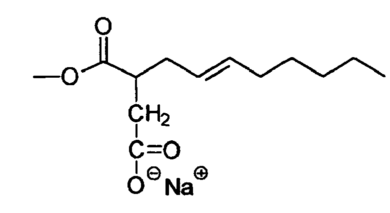 und welches z. B. unter der Handelsbezeichnung Amiogum^{®} 23 bei der Firma CERESTAR erhältlich ist sowie Aluminium Stärke Octenylsuccinate, insbesondere die unter den Handeisbezeichnungen Dry Flo^{®} Elite LL und Dry Flo^{®} PC bei der Firma CERESTAR erhältlichen.

Vorteilhaft ist es, den mittleren Partikeldurchmesser der verwendeten modifizierten Polysaccharide kleiner als 20 µm, besonders vorteilhaft kleiner als 15 µm zu wählen.

Die Liste der genannten modifizierten Polysaccharide, die erfindungsgemäße Pickering-Emulsionen stabilisieren können, soll selbstverständlich nicht limitierend sein. Modifizierte Polysaccharide im Sinne der vorliegenden Erfindung sind auf zahlreichen, an sich bekannten Wegen, sowohl chemischer als auch physikalischer Natur erhältlich.

Die vorstehend genannten amphiphilen Partikel eignen sich hervorragend sowohl zur Stabilisierung von W/O-Pickering-Emulsionen als auch zur Stabilisierung von O/W-Pikkering-Emulsionen. Nachfolgend werden erfindungsgemäße mikrofeine Partikel genannt, welche vorteilhaft insbesondere einen der beiden Emulsionstypen W/O bzw. O/W stabilisieren.

### W/O-Pickering-Emulsionen:

Der Wasserphasenanteil der erfindungsgemäßen W/O-Pickering-Emulsionen wird vorzugsweise aus dem Bereich von 0,5 bis 75 Gew. % gewählt, bezogen auf das Gesamtgewicht der Formulierungen.

Vorteilhaft zur Stabilisierung von W/O-Pickering-Emulsionen sind insbesondere auch Magnesiumsilicate (auch: Talkum), beispielsweise die unter der Handelsbezeichnung Talkum Micron bei der Firma Grolmann erhältlichen.

### O/W-Pickering-Emulsionen:

Der Fettphasenanteil der erfindungsgemäßen OIW-Pickering-Emulsionen wird vorzugsweise aus dem Bereich von 0,5 bis 75 Gew. % gewählt, bezogen auf das Gesamtgewicht der Formulierungen.

Besonders vorteilhaft zur Stabilisierung von O/W-Pickering-Emulsionen sind im Sinne der vorliegenden Erfindung auch unbehandelte, nahezu reine Pigmentpartikel, beispielsweise Titandioxid-Pigmente, insbesondere solche, die unter der Handelsbezeichnung KRONOS^{®} 1171 (TiO₂) von der Firma Kronos Titan erhältlich sind.

O/W-Pickering-Emulsionen werden im Sinne der vorliegenden Erfindung ferner besonders vorteilhaft durch Metalloxidpartikel stabilisiert, die mit Aluminiumhydroxid und/oder Siliziumdioxid überzogen ("gecoatet") sind. Vorteilhafte Ausführungsformen sind beispielsweise Titandioxidpartikel, die unter dem Namen EUSOLEX^{®} TA bei der Firma Merck erhältlich sind.

Es ist ferner vorteilhaft, wenngleich nicht zwingend, die erfindungsgemäßen mikrofeinen Partikel mit weiteren amphiphilen Partikeln zu kombinieren, welche gegebenenfalls auch zur Stabilisierung der Pickering-Emulsionen beitragen können.

Solche Partikel sind beispielsweise Titandioxidpigmente, die mit Octylsilanol beschichtet sind, und/oder Siliciumdioxidpartikel, die oberflächlich wasserabweisend behandelt sind. Geeignete Siliciumdioxidpartikel sind beispielsweise sphärische Polyalkylsilsesquioxan-Partikel, wie sie in der Europäischen Offenlegungsschrift 0 686 391 erwähnt werden. Solche Polyalkylsilsesquioxan-Partikel sind beispielsweise unter den Handelsbezeichnungen Aerosil R972 und Aerosil 200V bei der Firma Degussa erhältlich. Geeignete Titandioxidpartikel sind unter der Handelsbezeichnung T805 ebenfalls bei der Firma Degussa erhältlich.

Die erfindungsgemäßen Pickering-Emulsionen können als Grundlage für kosmetische oder dermatologische Formulierungen dienen. Diese können wie üblich zusammengesetzt sein und beispielsweise zur Behandlung und der Pflege der Haut, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Erfindungsgemäße Pickering-Emulsionen können auch Verdickungsmittel enthalten, um die taktilen Eigenschaften der Emulsion zu verbessern.

Insbesondere können die erfindungsgemäßen Pickering-Emulsionen auch Antioxidantien enthalten. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B.

Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO₄) Seien und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Günstig sind auch kosmetische und dermatologische Zubereitungen, die in der Form eines Sonnenschutzmittels vorliegen. Vorzugsweise enthalten diese mindestens eine UV-A-Filtersubstanz und/oder mindestens eine UV-B-Filtersubstanz und/oder mindestens ein weiteres anorganisches Pigment aus der Gruppe der Oxide des Eisens, Zirkoniums, Siliciums, Mangans, Aluminiums, Cers und Mischungen davon sowie Abwandlungen, bei denen die Oxide die aktiven Agentien sind.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A-bzw. UV-B-Filtersubstanzen eingearbeitet.

Vorteilhaft können erfindungsgemäße Zubereitungen Substanzen enthalten, die UV-Strahlung im UV-B-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Enthalten die erfindungsgemäßen Emulsionen UV-B-Filtersubstanzen, können diese öllöslich oder wasserlöslich sein. Erfindungsgemäß vorteilhafte öllösliche UV-B-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoäsäure(2-ethylhexyl)ester, 4-(Dimethylamino)benzoäsäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- hinsichtlich der C₃-Achse des Triazingrundkörpers symmetrisch Triazinderivate, vorzugsweise 4,4',4"-(1,3,5-Triazin-2,4,6-triyltriimino)-tris-benzoesäure-tris(2-ethylhexylester),
- Benzotriazolderivate, vorzugsweise 2,2'-Methylen-bis-(6-(2H-benzotriazol-2-yl)-4-(1,1,3,3-tetramethylbutyl)-phenol)
- sowie an Polymere gebundene UV-Filter.

Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UV-B-Filter, die in den erfindungsgemäßen Pickering-Emulsionen verwendet werden können, soll selbstverständlich nicht limitierend sein.

Es kann auch von Vorteil sein, in erfindungsgemäßen Pickering-Emulsionen UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Weitere vorteilhafte UV-A-Filtersubstanzen sind die Phenylen-1,4-bis-(2-benzimidazyl)-3,3'-5,5'-tetrasulfonsäure: 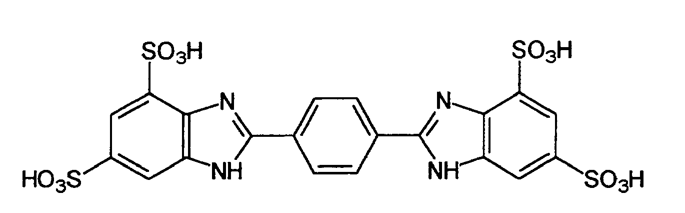 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz: 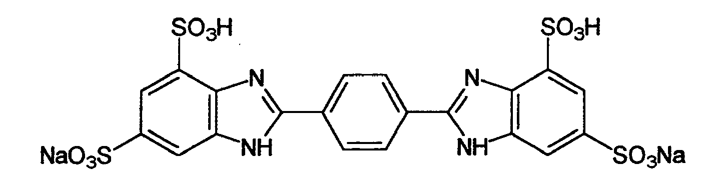 sowie das 1,4-Di(2-oxo-10-sulfo-3-bomylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-suifonsäure) bezeichnet wird und sich durch die folgende Struktur auszeichnet:

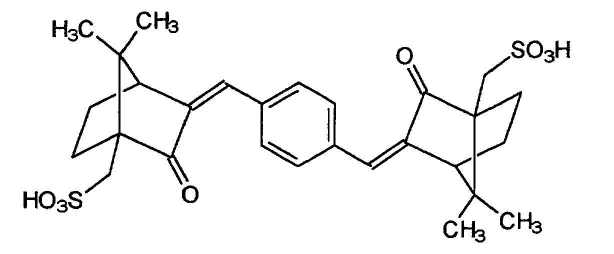

Auch Zubereitungen, die UV-A-Filter enthalten, sind Gegenstand der Erfindung. Es können die für die UV-B-Kombination verwendeten Mengen eingesetzt werden.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, llit, Beidellit, Nontronit, Saponit, Hektorit, Bentonit, Smectit, ferner beispielsweise Zinksalze der Ricinolsäure. Keimhemmende Mittel sind ebenfalls geeignet, in die erfindungsgemäßen W/O-Emulsionsstifte eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2'6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Liste der genannten Wirkstoffe bzw. Wirkstoffkombinationen soll selbstverständlich nicht limitierend sein.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

| | | **1** | **2** | **3** | **4** | **5** |
|---|---|---|---|---|---|---|
| | **W/O** | **W/O** | **W/O** | **O/W** | **O/W** | **O/W** |
| Titandioxid (Eusolex T2000) | 2 | 4 | 6 | 3 | 5 | 2 |
| Zinkoxid | 5 | | 4 | | | 4 |
| Titandioxid (Titandioxid T805) | | | 2 | | | |
| Silica (Aerosil R972) | | 1 | 0,5 | | | |
| Talkum (Talkum Micron) | | 0,5 | | | | |
| Bomitrid | | 2 | | | | |
| Natrium Maisstärke n-Octenylsuccinat | | | | 0,5 | | 1 |
| Hydroxystearylhydroxystearat (Elfacos C26) | 2 | | 2 | | | |
| C₂₀₋₄₀ Alkylstearat (Kesterwachs K82) | 1 | 1 | | | | 2 |
| C₁₆₋₃₈ AlkylhHydroxystearoylstearat (Kesterwachs K80P) | | 2 | | 3 | | 5 |
| Behenoxy Dimethicon (Abil Wax 2440) | | | 5 | | 5 | |
| Polyisobuten (Rewopal PIB 1000) | 5 | | | | | |
| CapryliclCapric Triglycerid | 5 | 5 | 5 | 20 | 20 | 20 |
| Octyldodecanol | 5 | | 5 | 15 | | 15 |
| Mineralöl | 10 | | | 10 | | 20 |
| Butylenglykol Caprylat/Caprat | | 10 | 10 | | 20 | 7 |
| C₁₂₋₁₅ Alkylbenzoat | 10 | 10 | 10 | 5 | 15 | |
| Dimethicon | | 2 | 3 | | | |
| Dicaprylyl Ether (Cetiol OE) | | | | 5 | | |
| Hydriertes Polyisobuten (Polysynlan) | 2 | | | | | |
| Methylbenzyliden Campher | | 3 | | | 4 | |
| Octyltriazon | | 1 | | | 4 | |
| Dibenzoylmethan | | 2 | | | 2 | |
| Dioctyl Butamido Triazon (UVASORB HEB) | | 2 | | | | |
| Konservierung | 0,5 | 0,5 | 0,5 | 0,5 | 0,5 | 0,5 |
| Glycerin | 5 | 10 | 3 | 5 | 5 | 5 |
| Biosaccharid Gel (Fucogel 1000) | | | 5 | | | |
| Hyaluronsäure | | | | | 0,5 | |
| NaCl | 1 | | 1 | | | |
| MgSO₄ | | 0,5 | | | | |
| Phenylbenzimiazol Sulfonsäure | | 1 | | | 2 | |
| Carbomer (Carbopol 981) | | | | 0,1 | | |
| Xanthan Gum | | | | 0,3 | | |
| Cellulose Gum (Natrosol Plus 330 CS) | | | 0,1 | | | |
| NaOH 45% | | 0,3 | | 0,1 | 0,7 | |
| EDTA-Lösung | | 1 | | | 1 | |
| Wasser | ad 100 | ad 100 | ad 100 | ad 100 | ad 100 | ad 100 |

## Patentansprüche

1. Pickering-Emulsionen, welche feindisperse Systeme vom Typ Wasser-in-Öl oder Öl-in-Wasser darstellen, enthaltend
(1) eine Ölphase, die mindestens ein Wachs enthält, welches aus der Gruppe der Triglyceride gewählt wird,
(2) eine Wasserphase,
(3) mindestens einen Typ mikrofeiner Partikel, die
a) eine mittlere Partikelgröße von weniger als 200 nm haben, die
b) sowohl hydrophile als auch lipophile Eigenschaften zeigen, welche also amphiphilen Charakter besitzen und sowohl in Wasser als auch in Öl dispergierbar sind und die
c) gegebenenfalls oberflächlich beschichtet sind, und
(4) höchstens 0,5 Gew.-% eines oder mehrerer Emulgatoren.

2. Pickering-Emulsionen nach Anspruch 1, **dadurch gekennzeichnet, daß** sie emulgatorfrei sind.

3. Pickering-Emulsionen nach einem der vorhergehenden Ansprüche, **dadurch gekennzeichnet, daß** der Gehalt der verwendeten Partikel zwischen 0,1 Gew.-% und 30 Gew.-% ist, bezogen auf das Gesamtgewicht der Zubereitungen.

4. Pickering-Emulsionen nach einem der vorhergehenden Ansprüche, **dadurch gekennzeichnet, daß** der Partikeldurchmesser der verwendeten Partikel zwischen 5 nm und 100 nm liegt.

5. Pickering-Emulsionen nach einem der vorhergehenden Ansprüche, **dadurch gekennzeichnet, daß** die verwendeten Partikel gewählt werden aus der Gruppe bestehend aus amphiphilen Metalloxiden, Bornitrid, mikrofeinen Polymerpartikeln und modifizierten Polysacchariden, wobei die Partikel sowohl einzeln als auch im Gemisch vorliegen können.

6. Pickering-Emulsionen nach einem der vorhergehenden Ansprüche, **dadurch gekennzeichnet, daß** die verwendeten Partikel oberflächlich wasserabweisend behandelt sind, wobei der amphiphile Charakter der Partikel gebildet wird bzw. erhalten bleibt.

7. Pickering-Emulsionen nach einem der vorhergehenden Ansprüche, **dadurch gekennzeichnet, daß** das oder die Triglyceride aus der Gruppe C₁₆₋₁₈ Triglycerid, Glycerylhydroxystearat, Hydrierte Coco-Glyceride, Caprylic/Capric/lsostearic/Adipic Triglycerid, C₁₈₋₃₆ Triglycerid, Glyceryltribehenat, Glyceryl-tri-(12-hydroxystearat), hydriertes Ricinusöl, C₁₆₋₂₄ Triglycerid gewählt wird.

8. Pickering-Emulsionen nach einem der vorhergehenden Ansprüche, **dadurch gekennzeichnet, daß** die Gesamtmenge an einem oder mehreren Wachsen in den fertigen kosmetischen oder dermatologischen Zubereitungen aus dem Bereich von 0,5 bis 20,0 Gew.-%, bevorzugt zwischen 1,0 und 5,0 Gew.-% gewählt wird, bezogen auf das Gesamtgewicht der Zubereitungen.

9. Pickering-Emulsionen nach einem der vorhergehenden Ansprüche, **dadurch gekennzeichnet, daß** die Wachse zusammen mit den anderen Ölkomponenten der Zubereitungen eine bei Raumtemperatur pastöse und streichfähige Masse bilden, welche bei 20 °C eine Viskosität von mehr als 5000 meer s aufweist

10. Pickering-Emulsionen nach einem der vorhergehenden Ansprüche, **dadurch gekennzeichnet, daß** sie Hilfsstoffe enthalten, die zur Verminderung oder Verhinderung eines stumpfen oder trockenen Hautgefühls nach deren Auftragen beitragen können.

## Claims

1. Pickering emulsions, which are finely disperse systems of the water-in-oil or oil-in-water type, comprising
(1) an oil phase, which comprises at least one wax which is chosen from the group of triglycerides,
(2) a water phase,
(3) at least one type of microfine particles which
a) have an average particle size of less than 200 nm, which
b) display both hydrophilic and lipophilic properties, i.e. which have amphiphilic character, and are dispersible both in water and in oil and which
c) have optionally been coated on the surface,
(4) at most 0.5% by weight of one or more emulsifiers.

2. Pickering emulsions according to Claim 1, **characterized in that** they are emulsifier-free.

3. Pickering emulsions according to one of the preceding claims, **characterized in that** the content of the particles used is between 0.1% by weight and 30% by weight, based on the total weight of the preparations.

4. Pickering emulsions according to one of the preceding claims, **characterized in that** the particle diameter of the particles used is between 5 nm and 100 nm.

5. Pickering emulsions according to one of the preceding claims, **characterized in that** the particles used are chosen from the group consisting of amphiphilic metal oxides, boron nitride, microfine polymer particles and modified polysaccharides, it being possible for the particles to be present individually or as a mixture.

6. Pickering emulsions according to one of the preceding claims, **characterized in that** the particles used have been surface-treated to repel water, where the amphiphilic character of the particles is formed or retained.

7. Pickering emulsions according to one of the preceding claims, **characterized in that** the triglyceride or triglycerides is/are chosen from the group consisting of C₁₆₋₁₈ triglyceride, glyceryl hydroxystearate, hydrogenated coco-glycerides, caprylic/capric/isostearic/adipic triglyceride, C₁₈₋₃₆ triglyceride, glyceryl tribehenate, glyceryl tri(12-hydroxystearate), hydrogenated castor oil, C₁₆₋₂₄ triglyceride.

8. Pickering emulsions according to one of the preceding claims, **characterized in that** the total amount of one or more waxes in the finished cosmetic or dermatological preparations is chosen from the range from 0.5 to 20.0% by weight, preferably between 1.0 and 5.0% by weight, based on the total weight of the preparations.

9. Pickering emulsions according to one of the preceding claims, **characterized in that** the waxes, together with the other oil components of the preparations, form a mass that is pasty and spreadable at room temperature which has a viscosity of more than 5000 mPa·s at 20°C.

10. Pickering emulsions according to one of the preceding claims, **characterized in that** they comprise auxiliaries which can contribute to reducing or preventing a rough or dry feel on the skin following their application.

## Revendications

1. Emulsions de Pickering, qui représentent des systèmes finement dispersés du type eau-dans-huile ou huile-dans-eau, contenant
(1) une phase huileuse, qui comprend au moins une cire qui est choisie dans le groupe des triglycérides,
(2) une phase aqueuse,
(3) au moins un type de particules microfines qui
a) ont une taille moyenne de particule inférieure à 200 nm, qui
b) présentent aussi bien des propriétés hydrophiles que des propriétés lipophiles, qui ont donc un caractère amphiphile et sont dispersables aussi bien dans l'eau que dans l'huile et qui
c) sont éventuellement enrobées en surface, et
(4) au maximum 0,5 % en poids d'un ou plusieurs émulsifiants.

2. Emulsions de Pickering selon la revendication 1, **caractérisées en ce qu'**elles sont exemptes d'émulsifiant.

3. Emulsions de Pickering selon l'une quelconque des revendications précédentes, **caractérisées en ce que** la teneur en les particules utilisées est comprise entre 0,1 % en poids et 30 % en poids, par rapport au poids total des préparations.

4. Emulsions de Pickering selon l'une quelconque des revendications précédentes, **caractérisées en ce que** le diamètre de particule des particules utilisées est compris entre 5 nm et 100 nm.

5. Emulsions de Pickering selon l'une quelconque des revendications précédentes, **caractérisées en ce que** les particules utilisées sont choisies dans le groupe constitué par des oxydes métalliques amphiphiles, le nitrure de bore, des particules de polymère microfines et des polysaccharides modifiés, les particules pouvant être présentes aussi bien individuellement qu'en mélange.

6. Emulsions de Pickering selon l'une quelconque des revendications précédentes, **caractérisées en ce que** les particules utilisées présentent en surface un traitement hydrofuge, le caractère amphiphile des particules étant acquis ou conservé.

7. Emulsions de Pickering selon l'une quelconque des revendications précédentes, **caractérisées en ce que** le triglycéride ou les triglycérides est/sont choisi(s) dans le groupe constitué par un triglycéride en C₁₆₋₁₈, l'hydroxystéarate de glycéryle, les glycérides de coco hydrogénés, un triglycéride caprylique/caprique/isostéarique/adipique, un triglycéride en C₁₈₋₃₆, le tribéhénate de glycéryle, le glycéryl-tri(12-hydroxystéarate), l'huile de ricin hydrogénée, un triglycéride en C₁₆₋₂₄.

8. Emulsions de Pickering selon l'une quelconque des revendications précédentes, **caractérisées en ce que** la quantité totale d'une ou de plusieurs cires dans les préparations cosmétiques ou dermatologiques finales est choisie dans la plage allant de 0,5 à 20,0 % en poids, de préférence entre 1,0 et 5,0 % en poids, par rapport au poids total des préparations.

9. Emulsions de Pickering selon l'une quelconque des revendications précédentes, **caractérisées en ce** les cires, conjointement avec les autres composants huileux des préparations, forment une masse pâteuse et pouvant être étalée à température ambiante qui présente une viscosité supérieure à 5000 mPa.s à 20°C.

10. Emulsions de Pickering selon l'une quelconque des revendications précédentes, **caractérisées en ce qu'**elles comprennent des adjuvants qui peuvent contribuer à diminuer ou à empêcher une sensation rêche ou sèche sur la peau suite à leur application.
